# EUROPÄISCHE PATENTSCHRIFT

(11) **EP 0 942 920 B1**
(45) Veröffentlichungstag und Bekanntmachung des Hinweises auf die Patenterteilung: **30.01.2002**
(21) Anmeldenummer: 97954725.4
(22) Anmeldetag: 08.12.1997
(51) Int. Cl.: C07J 53/00, A61K 31/565

(54) **STEROIDESTER, VERFAHREN ZU IHRER HERSTELLUNG UND IHRE PHARMAZEUTISCHE VERWENDUNG**
STEROID ESTERS, METHOD FOR THE MANUFACTURE THEREOF AND THEIR PHARMACEUTICAL USE
ESTERS STEROIDES, LEUR PROCEDE DE PREPARATION ET LEUR UTILISATION PHARMACEUTIQUE

(30) Priorität: 06.12.1996 DE 19652408
(43) Veröffentlichungstag der Anmeldung: 22.09.1999
(73) Patentinhaber: SCHERING AKTIENGESELLSCHAFT, 13353 Berlin (DE)
(72) Erfinder: KLAR, Ulrich, D-13503 Berlin (DE); CLEVE, Arwed, D-10707 Berlin (DE); SCHWEDE, Wolfgang, D-13467 Berlin (DE); NEEF, Günter, D-10711 Berlin (DE); OTTOW, Eckhard, D-12203 Berlin (DE); CHWALISZ, Kristof, D-13503 Berlin (DE); SCHNEIDER, Martin, D-13469 Berlin (DE)
(86) Internationale Anmeldenummer: EP9706829
(87) Internationale Veröffentlichungsnummer: WO9824803

(56) Entgegenhaltungen:
- EP-A- 0 283 428
- WO-A-96/19997
- DE-A- 4 434 488

## Beschreibung

Die Erfindung betrifft Steroidester, Verfahren zu ihrer Herstellung sowie ihre Verwendung als Hilfsstoffe für pharmakologische Untersuchungen und als Arzneistoffe.

Die Erfindung betrifft Steroidester der Formel I 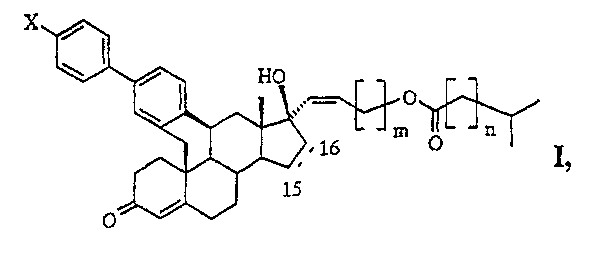 worin
- m: 1 oder 2
- n: 0 oder 1
- X: F oder CN und
die gestrichelte Linie zwischen den Kohlenstoffatomen 15 und 16 die etwaige Anwesenheit einer Doppelbindung symbolisiert,
ausgenommen die Verbindung (Z)-6'-(4-Cyanphenyl)-9,11α-dihydro-17ß-hydroxy-17α-[3-(1-oxo-2-methylpropoxy)-1-propenyl]-4'*H*-naphtho[3',2',1':10,9,11]estr-4-en-3-on.

Die nachstehenden Verbindungen sind erfindungsgemäß besonders bevorzugt:
(*Z*)-6'-(4-Cyanphenyl)-9,11α-dihydro-17β-hydroxy-17α-[3-(1-oxo-3-methylbutoxy)-1-propenyl]-4'*H*-naphtho[3',2',1':10,9,11]estr-4-en-3-on
(*Z*)-6'-(4-Cyanphenyl)-9,11α-dihydro-17β-hydroxy-17α-[4-(1-oxo-2-methylpropoxy)-1-butenyl]-4'*H*-naphtho[3',2',1':10,9,11]estr-4-en-3-on
(*Z*)-6'-(4-Cyanphenyl)-9,11α-dihydro-17β-hydroxy-17α-[4-(1-oxo-3-methylbutoxy)-1-butenyl]-4'*H*-naphtho[3',2',1':10,9,11]estr-4-en-3-on
(*Z*)-9,11α-Dihydro-6'-(4-fluorphenyl)-17β-hydroxy-17α-[4-(1-oxo-3-methylbutoxy)-1-butenyl]-4'*H*-naphtho[3',2',1':10,9,11]estr-4-en-3-on
(*Z*)-6'-(4-Cyanphenyl)-9,11α-dihydro-17β-hydroxy-17α-[3-(1-oxo-3-methylbutoxy)-1-propenyl]-4'*H*-naphtho[3',2',1':10,9,11]estra-4,15-dien-3-on
(*Z*)-6'-(4-Cyanphenyl)-9,11α-dihydro-17β-hydroxy-17α-[3-(1-oxo-2-methylpropoxy)-1-propenyl]-4'*H*-naphtho[3',2',1':10,9,11]estra-4,15-dien-3-on.

Viele steroidale Verbindungen, insbesondere solche mit 11-Arylrest sind aufgrund ihres Substitutionsmusters zum Teil sehr schlecht löslich, sodaß zum Erhalt therapeutisch relevanter Plasmaspiegel oftmals überproportional hohe Einzel- oder Mehrfachdosierungen oder aufwendige Formulierungstechniken angewendet werden müssen.

In der DE-A 44 34 488 ist beschrieben, daß die Löslichkeit derartiger Verbindungen erheblich verbessert werden kann, wenn an geeigneter Stelle im Molekül eine freie Hydroxylgruppe verestert oder mit einer Amidgruppe versehen wird. Die Alkanoyloxygruppe der Estergruppierung in der allgemeinen Formel weist dort in den strukturell nächstliegenden Fällen, d.h. im Fall eines 3-Keto-4-en-11β,19-[4-(4-Subst.-phenyl)-o-phenylen]-Steroids mit 17α-Alk-1-enyl-Seitenkette mindestens 6 Kohlenstoffatome auf. Außerdem ist als einzelne Verbindung (*Z*)-6'-(4-Cyanphenyl)-9,11α-dihydro-17β-hydroxy-17α-[3-(1-oxo-2-methylpropoxy)-1-propenyl]-4'*H*-naphtho[3',2',1':10,9,11]estr-4-en-3-on (Referenzverbindung 3) beschrieben.

Desweiteren sind 3-Keto-4-en-11β,19-[4-(4-Subst.-phenyl)-o-phenylen]-Steroide mit einer 17α-Alk-1-enyl-Seitenkette mit endständiger Hydroxyfunktion in der EP-A 0 283 428 beschrieben; die Hydroxygruppe kann mit einer Acylgruppe mit bis zu 4 Kohlenstoffatomen verestert sein. Spezielle Ester sind nicht beschrieben.

Die erfindungsgemäßen Verbindungen der allgemeinen Formel I fallen somit weder in den Rahmen der DE-A 44 34 488 noch sind sie in der EP-A 0 283 428 vorbeschrieben.

Nunmehr wurde gefunden, daß sich die Verbindungen der allgemeinen Formel I überraschenderweise sowohl hinsichtlich ihrer Wirkstärke als auch ihrer Selektivität, d.h. durch ihr gesamtes Wirkprofil, vom nächstliegenden Stand der Technik der DE-A 44 34 488 abheben.

Es hat sich gezeigt, daß überraschenderweise eine empirische Korrelation zwischen der (temperaturabhängigen) Löslichkeit von Steroidestern, wie sie beispielweise in der DE-A 44 34 488 sowie in den Beispielen 1 bis 6 der vorliegenden Anmeldung beschrieben sind, in einem Gemisch aus Benzylbenzoat / Rizinusöl und deren biologischer Wirksamkeit nach oraler Applikation besteht. Eine in diesem Lösemittelsystem erhöhte Löslichkeit führt in der Regel zu einer gesteigerten Wirksamkeit der Verbindungen nach oraler Applikation.

Die Erfindung betrifft ferner Verfahren zur Herstellung von Steroidestern der Formel I, bei dem man Verbindungen der Formel II, die eine freie Hydroxylgruppe tragen und worin m, X und die gestrichelte Linie zwischen den Kohlenstoffatomen 15 und 16 die in Formel I angegebenen Bedeutungen haben, mit einem Säureanhydrid der Formel III oder einem Säurechlorid der Formel IV, worin n den Wert 0 oder 1 besitzt, verestert.

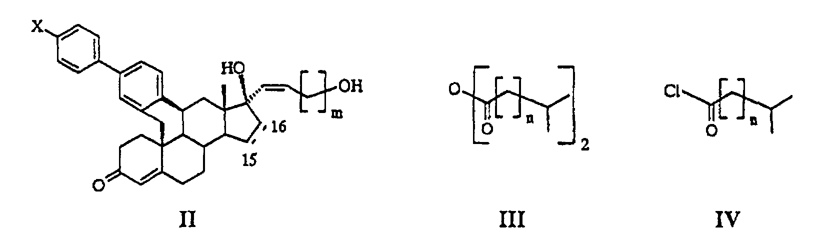

Die Veresterung erfolgt nach den dem Fachmann geläufigen Methoden, beispielsweise nach der in Beispiel 1 beschrieben Vorgehensweise.

### Löslichkeitsuntersuchungen der in den Beispielen 1 bis 6 genannten Ester

(1) (*Z*)-6'-(4-Cyanphenyl)-9,11α-dihydro-17β-hydroxy-17α-[3-(1-oxo-3-methylbutoxy)-1-propenyl]-4'*H*-naphtho[3',2',1':10,9,11]estr-4-en-3-on
(2) (*Z*)-6'-(4-Cyanphenyl)-9,11α-dihydro-17β-hydroxy-17α-[4-(1-oxo-2-methylpropoxy)-1-butenyl]-4'*H*-naphtho[3',2',1':10,9,11]estr-4-en-3-on
(3) (*Z*)-6'-(4-Cyanphenyl)-9,11α-dihydro-17β-hydroxy-17α-[4-(1-oxo-3-methylbutoxy)-1-butenyl]-4'*H*-naphtho[3',2',1':10,9,11]estr-4-en-3-on
(4) (*Z*)- 9,11α-Dihydro-6'-(4-fluorphenyl)-17β-hydroxy-17α-[4-(1-oxo-3-methylbutoxy)-1-butenyl)-4'*H*-naphtho[3',2',1':10,9,11]estr-4-en-3-on
(5) (*Z*)-6'-(4-Cyanphenyl)-9,11α-dihydro-17β-hydroxy-17α-[3-(1-oxo-3-methylbutoxy)-1-propenyl]-4'*H*-naphtho[3',2',1':10,9,11]estra-4,15-dien-3-on
(6) (*Z*)-6'-(4-Cyanphenyl)-9,11α-dihydro-17β-hydroxy-17α-[3-(1-oxo-2-methylpropoxy)-1-propenyl]-4'*H*-naphtho[3',2',1':10,9,11]estra-4,15-dien-3-on

In der folgenden Tabelle ist die Löslichkeit der Verbindungen 1 bis 6 im Vergleich zu der unveresterten Referenzverbindung 1 (*Z*)-6'-(4-Cyanphenyl)-9,11α-dihydro-17β-hydroxy-17α-(3-hydroxy-1-propenyl)-4'*H*-naphtho[3',2',1':10,9,11]estra-4-en-3-on (Beispiel 1 in DE-A 4216003) dargestellt. Für die Löslichkeit wird die Temperatur in °C angegeben, bei der sich 50 mg Substanz in 1 ml eines 1:4 Benzylbenzoat / Rizinusöl-Germisches lösen.
In allen Fällen ist die Löslichkeit gegenüber der unveresterten Verbindung signifikant verbessert.

| Verbindung | Zustand | Löslichkeit |
|---|---|---|
| ***Referenz- verbindung 1*** | ***kristallin*** | ***>> 100*** |
| ***Referenz- verbindung 3*** | ***kristallin*** | ***100*** |
| 1 | kristallin | 50 |
| 2 | kristallin | 23 |
| 3 | kristallin | 23 |
| 4 | kristallin | 50 |
| 5 | kristallin | 23 |
| 6 | kristallin | <50 |

### Untersuchungen zur Hydrolysestabilität der erfindungsgemäßen Ester

Die Hydrolysestabilität der Ester unter stark basischen Bedingungen kann als Einschätzung dafür dienen, ob die Verbindungen unter physiologischen Bedingungen eine ausreichende Stabilität besitzen.
Jeweils 10 mg der Verbindungen 1, 3 und 4 wurden bei 23°C in 10 ml Dioxan gelöst und mit 1 ml einer 1N wäßrigen Natronlauge versetzt. Es wurden jeweils 11 Proben gezogen, auf pH 6 angesäuert und per HPLC das Verhältnis Ester : Alkohol bestimmt. In der graphischen Darstellung ist der Anteil Ester in Prozent als Funktion der Verseifungsdauer in Stunden dargestellt.
Es zeigt sich, daß insbesondere die hier untersuchten Verbindungen 1, 3 und 4 selbst unter stark basischen Bedingungen eine sehr gute Stabilität aufweisen, die gegenüber der Referenzverbindung 3 signifikant verbessert ist.

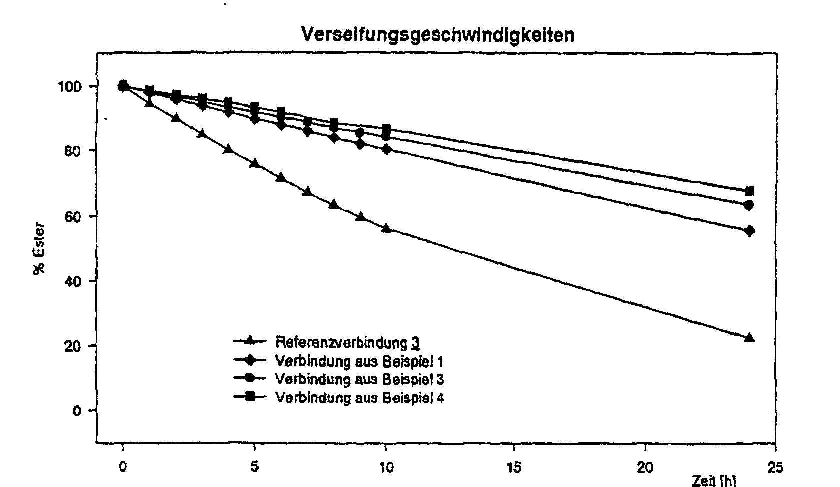

### Abortivtest an der Ratte als Maß der Progesteron-antagonisierenden Wirkung

### Versuchsdurchführung:

Als Tiermaterial dienen gravide Ratten im Gewicht von 190-220 g.
Die Tiere werden in Makrolonkäfigen in kontrolliert belichteten Räumen (10 Std. Dunkelheit, 14 Std. Helligkeit) bei einer Temperatur von 20°C gehalten, mit einer Standarddiät (pelletriertes Altromin) ernährt und mit Leitungswasser ad libitum getränkt.

### Galenische Zubereitung der Substanzen:

Die Testsubstanzen werden in einer Trägerflüssigkeit (85mg Myrj in 100ml 0,9% w/v Natriumchloridiösung) suspendiert und die Tagesdosis (siehe Tabelle) in einem Volumen von 0,5 ml oral appliziert.

### Versuchsansatz:

Die Ratten werden im Proestrus angepaart; der Beginn der Gravidität wird durch Nachweis von Spermien im Vaginalabstrich am Tag darauf - dies entspricht Tag 1 der Gravidität (d1 p.c.) - festgestellt. Die Tiere werden randomisiert und den einzelnen Dosierungs- bzw. der Kontrollgruppe zu je 4 bis 5 Tieren zugeordnet.
Vom 5. bis 7. Tag der Gravidität wird die Testsubstanz täglich verabreicht. Am 9. Tag werden Vaginalabstriche vorgenommen und die Tiere mit CO₂-Gas getötet.

### Auswertung:

Der Effekt der Behandlung wird durch Inspektion der Uteri untersucht. Die Rückbildung von implantaten und pathologische, hämorrhagische oder sonst abnorme Nidationsstellen werden als Abort gewertet.
Die Resultate des Rattenabortivtestes der Verbindungen 1 bis 6 sind in der folgenden Tabelle zusammengestellt. Die Referenzverbindung 2 ist (*Z*)-6'-(4-Cyanphenyl)-9,11α-dihydro-17α-[3-(3,3-dimethyl-1-oxobutoxy)-1-propenyl]-17β-hydroxy-4'*H*-naphtho [3', 2',1':10,9,11]est-4-en-3-on (Beispiel 7 in DE-A 44 34 488), die sich nur durch eine zusätzliche Methylgruppe in der Alkanoylgruppe des Esters von der Verbindung 1 der vorliegenden Anmeldung unterscheidet, sowie die bereits erwähnte Referenzverbindung 3.

| Abort/Kontrolle mg/d p.o. | Referenzverbindung | | Verbindung | | | | | |
|---|---|---|---|---|---|---|---|---|
| | 2 | 3 | 1 | 2 | 3 | 4 | 5 | 6 |
| 1,0 | 4/4 | nt | nt | nt | nt | nt | nt | nt |
| 0,3 | 2/4 | 5/5 | 5/5 | 4/4 | 4/4 | 4/4 | 4/4 | 4/4 |
| 0,1 | nt | 5/5 | 5/5 | 4/4 | 4/4 | 4/4 | 4/4 | 4/4 |
| 0,03 | nt | nt | 3/5 | 4/4 | 4/4 | 1/4 | 0/4 | 0/4 |
| 0,01 | nt | nt | 1/5 | 0/4 | 0/4 | nt | nt | nt |

| | | | | | | | | |
|---|---|---|---|---|---|---|---|---|
| nt: nicht getestet | | | | | | | | |

### Beeinflussung des Tumorwachstums im N-Methyl-N-nitrosoharnstoff (NMU) Modell bei der Ratte

### Biologische Grundlage:

Das Wachstum des NMU-induzierten Mammatumors der Ratte ist weitgehend vo Estrogenen und Gestagenen und weniger von Prolaktin abhängig. Estrogen- und Progesteronantagonisten führen zu einer Hemmung des Tumorwachstums.

### Tiermaterial:

Weibliche Ratten (Sprague-Dawley), 55 (± 3) Tage alt; pro Gruppe mind. 9 Tiere.

### Applikationsart:

Testsubstanz: p.o.
Vehikelvolumen p.o. = 0,1 ml/100g/d (0,9% NaCl/0,085% Myrj-53).
NMU i.v. = 50 mg/kg/d; 1,5ml/200g (0,9% NaCl/0,085% Myrj-53).

### Versuchsansatz:

Die Tiere erhalten einmalig 50 mg/kg NMU. Anschließend werden die Tiere einmal wöchentlich palpatorisch auf Tumorentwicklung untersucht. Ca. 6 bis 8 Wochen nach NMU-Behandlung entwickeln sich ein oder mehrere Tumore pro Tier. Bei einer Mindestgröße von 150 mm²/Tumor/Tier beginnt die Behandlung (6 mal pro Woche) mit der Testsubstanz, die Ermittlung des Körpergewichts und der Tumorgröße (mit Hilfe einer Schiebelehre; 1 mal pro Woche).
Die Resultate für die Verbindung 1 sind in den folgenden Abbildungen für unterschiedliche Dosierungen jeweils im Vergleich zur unbehandelten Kontrolle, einer Ovariektomiegruppe sowie der Referenzverbindung 3, (*Z*)-6'-(4-Cyanphenyl)-9,11α-dihydro-17β-hydroxy-17α-[3-(1-oxo-2-methylpropoxy)-1-propenyl]-4'*H* -naphtho[3',2',1':10,9,11]estr-4-en-3-on, graphisch dargestellt. Beide Versuche belegen ganz klar, daß die erfindungsgemäße Verbindung 1 eine gegenüber der Referenzverbindung 3 überlegene tumorhemmende Wirkung aufweist. Diese ist aufgrund der nur geringfügigen strukturellen Unterschiede überraschend.

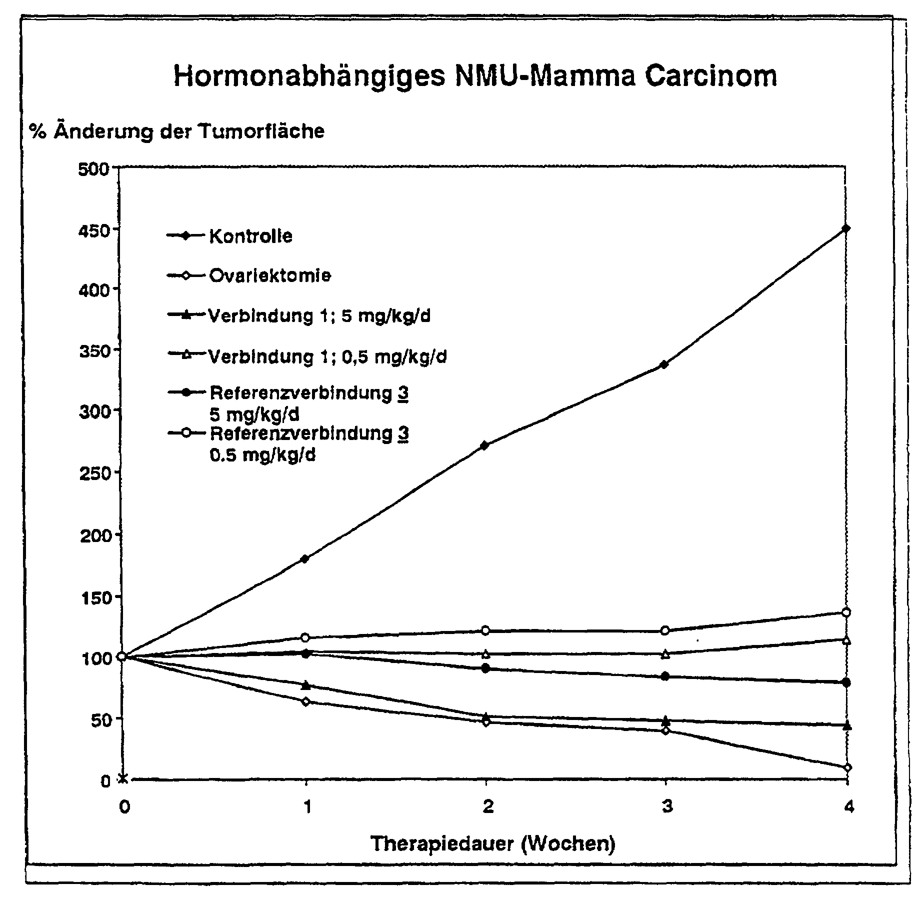

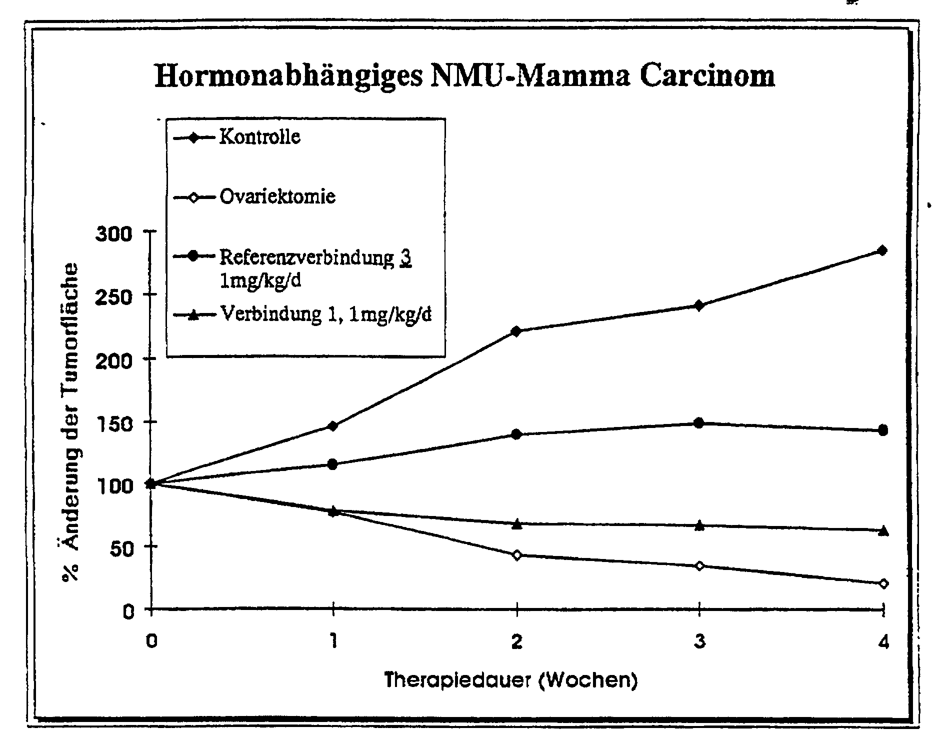

### Tumorhemmende Wirkung an der hormonsensitiven humanen Mammakarzinomzellinie ZR-75.

### Biologische Grundlage:

Die Mammakarzinomzellinie ZR-75 ist eine estrogen- und progesteronrezeptor-positive humane Linie, die seriell in der thymusaplastischen Nacktmaus transplantierbar ist. Sie spricht auf die Standardtherapie des Brustkrebses in der Klinik - Tamoxifen - mit einer Wachstumshemmung an. Eine hier verwendete Unterlinie entwickelte nach längerer Therapiedauer eine Tamoxifenresistenz.

### Tiermaterial:

Weibliche nu/nu Mäuse, 4 Wochen alt.

### Formulierung und Applikation der Testsubstanzen:

Die Testsubstanzen werden in Benzylbenzoat und Rizinusöl (1+4) gelöst und die einmalige Dosis in einem Volumen von 0.1 ml s.c. appliziert. Die Therapie beginnt ca. 7 Wochen nach Implantation (Therapie etablierter Tumoren). Die Behandlungszeit beträgt max. 10 Wochen.

### Versuchsansatz:

Die Tumorfragmente von mehreren Donortieren werden den Tieren s.c. beidseitig in die Flanken implantiert. Am Tag der Implantation und am Tag 60 werden alle Tiere mit einem Tamoxifen-freisetzenden Pellet (5 mg) implantiert.
Die Mäuse werden 7 Wochen nach Implantation der Tumoren randomisiert und die Therapie wird begonnen und über 8 Wochen durchgeführt.
Das Tumorwachstum wird durch Ermittlung der Tumorfläche mittels einer Schiebelehre bestimmt. Die Tumorfläche errechnet sich aus dem Produkt des längsten und des darauf senkrecht stehenden Durchmessers des Tumors. Zu Versuchsende werden die Tiere getötet, die Tumoren herauspräpariert und gewogen.

### Auswertung:

Die Hemmung des Wachstumsverlaufs der Tumoren ist in der folgenden Abbildung für die Verbindung aus Beispiel 1 im Vergleich zur unbehandelten Kontrollgruppe grafisch dargestellt.

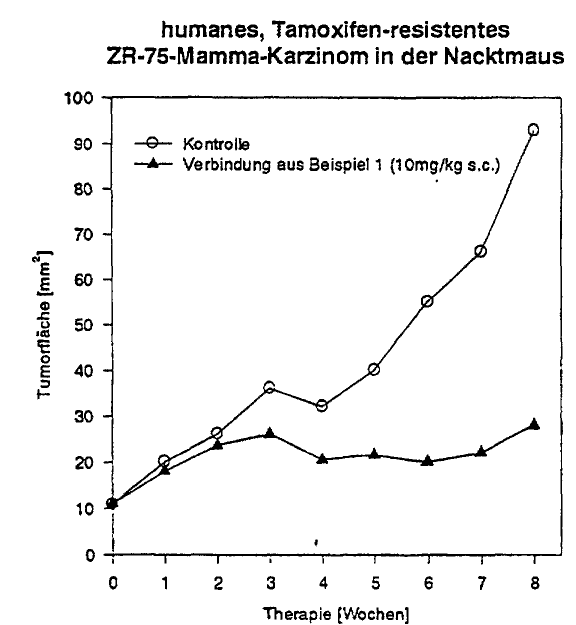

Das Ergebnis belegt, daß die bereits in *in vitro* Versuchen beobachtete sehr gute Antitumorwirkung auch auf die *in vivo* Situation übertragbar ist.

Die neuen Verbindungen der allgemeinen Formel I stellen somit wertvolle pharmazeutische Wirkstoffe dar. Sie verfügen über eine starke Affinität zum Gestagenrezeptor und besitzen starke antigestagene Eigenschaften. Diese wichtige biologische Wirksamkeit-kann für medizinische Zwecke genutzt werden.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.
Die erfindungsgemäßen Verbindungen der allgemeinen Formel I sind auch zur Herstellung von Präparaten für die Empfängnisverhütung für die Frau geeignet (WO-A 93/23020).
Sie können außerdem gegen hormonelle Unregelmäßigkeiten, zur Menstruationsauslösung und zur Geburtseinleitung eingesetzt werden. Weitere Indikationsgebiete im Bereich der Gynäkologie sind die Behandlung von den mit einer Dysmenorrhoe einhergehenden Beschwerden sowie der Endometriose.
Außerdem sind die erfindungsgemäßen Verbindungen bestens für die Behandlung von hormonabhängigen Carcinomen geeignet.

Die erfindungsgemäßen, antigestagen wirksamen Verbindungen der allgemeinen Formel I können auch mit antiestrogen wirksamen Verbindungen zur Herstellung pharmazeutischer Präparate zur Behandlung hormonabhängiger Tumoren (EP-A 0 310 542), zur Geburtseinleitung, zum Schwangerschaftsabbruch sowie zur Behandlung gynäkologischer Störungen (EP-A 0 310 541) und zur weiblichen Kontrazeption (WO 96/19997) verwendet werden.

Die Erfindung betrifft somit auch Arzneimittel auf Basis der pharmazeutisch verträglichen, d.h. in den verwendeten Dosen nicht toxischen Verbindungen der allgemeinen Formel I, gegebenenfalls in Verbindung mit einem Antiestrogen, zusammen mit den üblichen Hilfs- und Trägerstoffen.

Schließlich betrifft die vorliegende Erfindung auch die Verwendung der Verbindungen der allgemeinen Formel I, gegebenenfalls zusammen mit einem Antiestrogen, zur Herstellung von Arzneimitteln.

Die erfindungsgemäßen Verbindungen können nach an sich bekannten Methoden der Galenik zu pharmazeutischen Präparaten für die enterale, percutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen oder mittels intravaginaler (z.B. Vaginalringe) oder intrauterinen Sytemen (Pessare, Spiralen) verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens oder Myrj, Magnesiumstearat, wäßrigen oder nicht wäßrigen Trägern, Paraffinderivaten, Netz-, Dispergier-, Emulgier-, Konservierungsmitteln und Aromastoffen zur Geschmackskorrektur (z.B. etherischen Ölen) gemischt werden.

Eine Dosiseinheit enthält etwa 0,1-100 mg Wirkstoff(e). Die Dosierung der erfindungsgemäßen Verbindungen liegt beim Menschen bei etwa 0,1-1000 mg pro Tag.

Die nachfolgenden Beispiele dienen der näheren Erläuterung der vorliegenden Erfindung:

### BEISPIEL 1

### (Z)-6'-(4-Cyanphenyl)-9,11α-dihydro-17β-hydroxy-17α-[3-(1-oxo-3-methylbutoxy)-1-propenyl]-4'H-naphtho[3',2',1':10,9,11]estr-4-en-3-on

5,0g (9,62 mmol) (*Z*)-6'-(4-Cyanphenyl)-9,11α-dihydro-17β-hydroxy-17α-[3-hydroxy-1-propenyl]-4'*H*-naphtho[3',2',1':10,9,11]estr-4-en-3-on, dessen Herstellung ausführlich in DE-A 42 16 004 beschrieben ist, löst man bei 23°C unter einer Atmosphäre aus trockenem Argon in einem Gemisch aus 50 ml Dichlormethan (p.a.) und 30 ml Pyridin (p.a.). Anschließend versetzt man mit 1,99 ml (9,94 mmol) Isovaleriansäureanhydrid, 200 mg (1,64 mmol) 4-Dimethylaminopyridin und rührt 18h bei 23°C.
Man gießt in eine gesättigte Natriumhydrogencarbonatlösung, rührt 15 Minuten nach und extrahiert mehrfach mit Dichlormethan. Die vereinigten organischen Extrakte wäscht man mit gesättigter Natriumchloridlösung und trocknet über Natriumsulfat. Den nach Filtration und Lösungsmittelabzug erhaltenen Rückstand versetzt man mit Toluol, engt im Vakuum ein und reinigt durch Chromatographie an Aluminiumoxid der Aktivitätsstufe III mit einem Gemisch aus n-Hexan und Ethylacetat.
Isoliert werden 5,43g (8,99 mmol, 93,4%) der Titelverbindung als kristalliner Feststoff, den man bei 23°C in Ethylacetat löst und durch Zugabe von Diisopropylether langsam ausfällt. Nach Filtration und Trocknung werden 5,11g (8,46 mmol, 88,0%) reine Titelverbindung mit einem Schmelzpunkt von 174-176°C erhalten.
¹H-NMR (CDCl₃): δ= 0,50 (3H), 0,98 (6H), 1,16 (1H), 1,24-1,47 (4H), 1,65-2,34 (12H), 2,35-2,52 (2H), 2,58 (1H), 2,65 (1H), 3,08 (1H), 3,31 (1H), 3,36 (1H), 4,89 (1H), 5,14 (1H), 5,53 (1H), 5,72 (1H), 5,89 (1H), 7,32 (1H), 7,42 (1H), 7,55 (1H), 7,71 (4H) ppm.

### BEISPIEL 2

### (Z)-6'-(4-Cyanphenyl)-9,11α-dihydro-17β-hydroxy-17α-[4-(1-oxo-2-methylpropoxy)-1-butenyl]-4'H-naphtho[3',2',1':10,9,11]estr-4-en-3-on

1,50g (2,81 mmol) (*Z*)-6'-(4-Cyanphenyl)-9,11α-dihydro-17β-hydroxy-17α-[4-hydroxy-1-butenyl]-4'*H*-naphtho[3',2',1':10,9,11]estr-4-en-3-on, das man in Analogie zu den in DE-A 42 16 004 beschriebenen Verfahren hergestellt hat, setzt man in Analogie zu Beispiel 1 unter Verwendung von Isobuttersäureanhydrid um und isoliert nach Aufarbeitung und Reinigung 1,33g (2,20 mmol, 79%) der Titelverbindung als farblosen kristallinen Feststoff.
¹H-NMR (CDCl₃): δ= 0,51 (3H), 1,06-1,45 (4H), 1,19 (6H), 1,63-2,13 (9H), 2,28 (1H), 2,42 (2H), 2,53-2,73 (4H), 2,82 (1H), 2,93 (1H), 3,32 (1H), 3,36 (1H), 4,17 (2H), 5,50 (1H), 5,66 (1H), 5,89 (1H), 7,32 (1H), 7,42 (1H), 7,56 (1H), 7,71 (4H) ppm.

### BEISPIEL 3

### (Z)-6'-(4-Cyanphenyl)-9,11α-dihydro-17β-hydroxy-17α-[4(1-oxo-3-methylbutoxy)-1-butenyl]-4'H-naphtho[3',2',1':10,9,11]estr-4-en-3-on

1,5g (2,81 mmol) (*Z*)-6'-(4-Cyanphenyl)-9,11α-dihydro-17β-hydroxy-17α-[4-hydroxy-1-butenyl]-4'*H*-naphtho[3',2',1':10,9,11]estr-4-en-3-on, das man in Analogie zu den in DE-A 42 16 004 beschriebenen Verfahren hergestellt hat, setzt man in Analogie zu Beispiel 1 um und isoliert nach Aufarbeitung und Reinigung 1,30g (2,10 mmol, 75%) der Titelverbindung als farblosen kristallinen Feststoff.
¹H-NMR (CDCl₃): δ= 0,50 (3H), 0,98 (6H), 1,07-1,46 (4H), 1,56-2,35 (13H), 2,35-2,53 (2H), 2,53-2,73 (3H), 2,81 (1H), 2,90 (1H), 3,31 (1H), 3,35 (1H), 4,17 (2H), 5,50 (1H), 5,67 (1H), 5,90 (1H), 7,32 (1H), 7,42 (1H), 7,57 (1H), 7,71 (4H) ppm.

### BEISPIEL 4

### (Z)-9,11α-Dihydro-6'-(4-fluorphenyl)-17β-hydroxy-17α-[4-(1-oxo-3-methylbutoxy)-1-butenyl]-4'H-naphtho[3',2',1':10,9,11]estr-4-en-3-on

465mg (0,87 mmol) (Z)-9,11α-Dihydro-6'-(4-fluorphenyl)-17β-hydroxy-17α-[4-hydroxy-1-butenyl]-4'*H*-naphtho[3',2',1':10,9,11]estr-4-en-3-on, das man in Analogie zu den in DE-A 42 16 004 beschriebenen Verfahren hergestellt hat, setzt man in Analogie zu Beispiel 1 um und isoliert nach Aufarbeitung und Reinigung 512mg (0,84 mmol, 96%) der Titelverbindung als farblosen kristallinen Feststoff.
¹H-NMR (CDCl₃): δ= 0,51 (3H), 0,98 (6H), 1,15 (1H), 1,23-1,45 (3H), 1,63-2,33 (13H), 2,33-2,54 (2H), 2,54-2,73 (3H), 2,80 (1H), 2,91 (1H), 3,31 (1H), 3,35 (1H), 4,17 (2H), 5,50 (1H), 5,67 (1H), 5,88 (1H), 7,12 (2H), 7,26 (1H), 7,37 (1H), 7,50 (1H), 7,75 (2H) ppm.

### BEISPIEL 5

### (Z)-6'-(4-Cyanphenyl)-9,11α-dihydro-17β-hydroxy-17α-[3-(1-oxo-3-methylbutoxy)-1-propenyl]-4'H-naphtho[3',2',1':10,9,11]estra-4,15-dien-3-on

50mg (97 µmol) (*Z*)-6'-(4-Cyanphenyl)-9,11α-dihydro-17β-hydroxy-17α-[3-hydroxy-1-propenyl]-4'*H*-naphtho[3',2',1':10,9,11]estra-4,15-dien-3-on, das man in Analogie zu den in DE-A 42 16 004 beschriebenen Verfahren hergestellt hat, setzt man in Analogie zu Beispiel 1 um und isoliert nach Aufarbeitung und Reinigung 47mg (78 µmol, 80%) der Titelverbindung als farblosen kristallinen Feststoff.
¹H-NMR (CDCl₃): δ= 0,52 (3H), 0,99 (6H), 1,24 (1H), 1,42 (1H), 1,73 (1H), 1,90 (1H), 1,98-2,35 (8H), 2,35-2,53 (2H), 2,53-2,75 (2H), 2,82 (1H), 3,10 (1H), 3,32 (1H), 3,40 (1H), 5,02 (2H), 5,61 (2H), 5,69 (1H), 5,89 (2H), 7,31 (1H), 7,42 (1H), 7,52 (1H), 7,70 (4H) ppm.

### BEISPIEL 6

### (Z)-6'-(4-Cyanphenyl)-9,11α-dihydro-17β-hydroxy-17α-[3-(1-oxo-2-methylpropoxy)-1-propenyl]-4'H-naphtho[3',2',1':10,9,11]estra-4,15-dien-3-on

50mg (97 µmol) (*Z*)-6'-(4-Cyanphenyl)-9,11α-dihydro-17β-hydroxy-17α-[3-hydroxy-1-propenyl]-4'*H*-naphtho[3',2',1':10,9,11]estra-4,15-dien-3-on, das man in Analogie zu den in DE-A 42 16 004 beschriebenen Verfahren hergestellt hat, setzt man in Analogie zu Beispiel 1 unter Verwendung von Isobuttersäureanhydrid um und isoliert nach Aufarbeitung und Reinigung 31mg (53 µmol, 55%) der Titelverbindung als farblosen kristallinen Feststoff.
¹H-NMR (CDCl₃): δ= 0,53 (3H), 1,20 (6H), 1,26 (1H), 1,43 (1H), 1,73 (1H), 1,91 (1H), 2,00-2,21 (4H), 2,28 (1H), 2,44 (2H), 2,53-2,75 (3H), 2,81 (1H), 3,08 (1H), 3,32 (1H), 3,41 (1H), 5,01 (2H), 5,62 (2H), 5,70 (1H), 5,90 (2H), 7,31 (1H), 7,42 (1H), 7,52 (1H), 7,70 (4H) ppm.

## Patentansprüche

1. Steroidester der Formel I 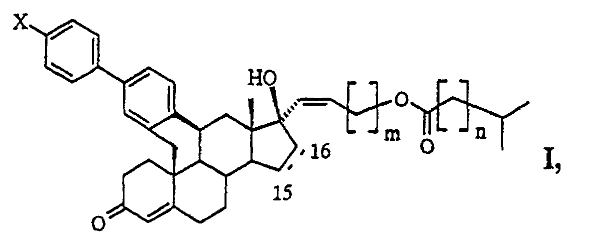 worin
m 1 oder 2
n 0 oder 1
X F oder CN und
die gestrichelte Linie zwischen den Kohlenstoffatomen 15 und 16 die etwaige Anwesenheit einer Doppelbindung symbolisiert,
ausgenommen die Verbindung (*Z*)-6'-(4-Cyanphenyl)-9,11α-dihydro-17β-hydroxy-17α-[3-(1-oxo-2-methylpropoxy)-1-propenyl]-4'*H*-naphtho[3',2',1':10,9,11]estr-4-en-3-on.

2. (*Z*)-6'-(4-Cyanphenyl)-9,11α-dihydro-17β-hydroxy-17α-[3-(1-oxo-3-methylbutoxy)-1-propenyl]-4'*H*-naphtho[3',2',1':10,9,11]estr-4-en-3-on
(*Z*)-6'-(4-Cyanphenyl)-9,11α-dihydro-17β-hydroxy-17α-[4-(1-oxo-2-methylpropoxy)-1-butenyl]-4'*H*-naphtho[3',2',1':10,9,11]estr-4-en-3-on
(*Z*)-6'-(4-Cyanphenyl)-9,11α-dihydro-17β-hydroxy-17α-[4-(1-oxo-3-methylbutoxy)-1-butenyl]-4'*H*-naphtho[3',2',1':10,9,11]estr-4-en-3-on
(*Z*)- 9,11α-Dihydro-6'-(4-fluorphenyl)-17β-hydroxy-17α-[4-(1-oxo-3-methylbutoxy)-1-butenyl]-4'*H*-naphtho[3',2',1':10,9,11]estr-4-en-3-on
(*Z*)-6'-(4-Cyanphenyl)-9,11α-dihydro-17β-hydroxy-17α-[3-(1-oxo-3-methylbutoxy)-1-propenyl]-4'*H-*naphtho[3',2',1':10,9,11]estra-4,15-dien-3-on
(*Z*)-6'-(4-Cyanphenyl)-9,11α-dihydro-17β-hydroxy-17α-[3-(1-oxo-2-methylpropoxy)-1-propenyl]-4'*H*-naphtho[3',2',1':10,9,11]estra-4,15-dien-3-on

3. Verbindungen nach Anspruch 1, worin m den Wert 1 besitzt.

4. Verbindungen nach Anspruch 1, worin m den Wert 2 besitzt.

5. Verbindungen nach Anspruch 1, worin n den Wert 1 besitzt.

6. Verbindungen nach Anspruch 1, worin n den Wert 0 besitzt.

7. Verbindungen nach Anspruch 1, worin X für eine Nitrilgruppe steht.

8. Verbindungen nach Anspruch 1, worin X für ein Fluoratom steht.

9. Verbindungen nach Anspruch 1, worin sich zwischen den Kohlenstoffatomen 15 und 16 eine Einfachbindung befindet.

10. Verbindungen nach Anspruch 1, worin sich zwischen den Kohlenstoffatomen 15 und 16 eine Doppelbindung befindet.

11. Verfahren zur Herstellung von Steroidestern der allgemeinen Formel I gemäß Anspruch 1, **dadurch gekennzeichnet, daß** man Verbindungen der Formel II, die eine freie Hydroxylgruppe tragen und worin m, X und die gestrichelte Linie zwischen den Kohlenstoffatomen 15 und 16 die in Formel I angegebenen Bedeutungen haben, mit einem Säureanhydrid der Formel III oder einem Säurechlorid der Formel IV, worin n den Wert 0 oder 1 besitzt, verestert

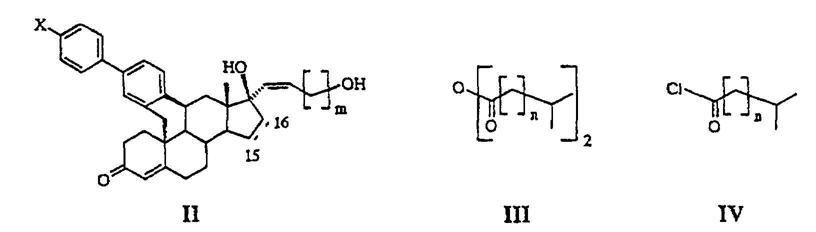

12. Arzneimittel, enthaltend mindestens eine Verbindung der allgemeinen Formel I gemäß Anspruch 1 sowie einen pharmazeutisch vertäglichen Träger.

13. Verwendung der Verbindungen der allgemeinen Formel I gemäß Anspruch 1 zur Herstellung von Arzneimitteln.

## Claims

1. A steroid ester of formula I 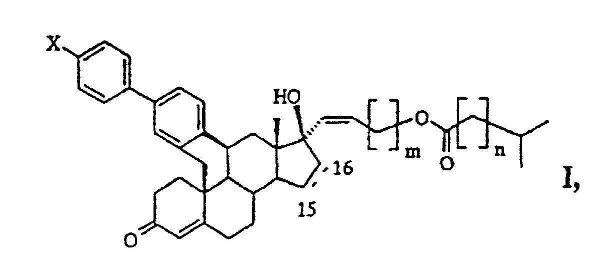 in which
m symbolizes 1 or 2
n symbolizes 0 or 1
x symbolizes F or CN and
the dotted line between carbon atoms 15 and 16 symbolizes the possible presence of a double bond, excluding the compound (Z)-6'-(4-cyanophenyl)-9,11α-dihydro-17β-hydroxy-17α-[3-(1-oxo-2-methylpropoxy)-1-propenyl]-4'H-naphtho[3',2',1':10,9,11]estr-4-en-3-one.

2. (Z)-6'-(4-Cyanophenyl)-9,11α-dihydro-17β-hydroxy-17α-[3-(1-oxo-3-methylbutoxy)-1-propenyl]-4'H-naphtho-[3',2',1':10,9,11]estr-4-en-3-one
(Z)-6'-(4-cyanophenyl)-9,11α-dihydro-17β-hydroxy-17α-[4-(1-oxo-2-methylpropoxy)-1-butenyl]-4'H-naphtho-[3',2',1':10,9,11]estr-4-en-3-one
(Z)-6'-(4-cyanophenyl)-9,11α-dihydro-17β-hydroxy-17α-[4-(1-oxo-3-methylbutoxy)-1-butenyl]-4'H-naphtho-[3',2',1':10,9,11]estr-4-en-3-one
(Z)-9,11α-dihydro-6'-(4-fluorophenyl)-17β-hydroxy-17α-[4-(1-oxo-3-methylbutoxy)-1-butenyl]-4'H-naphtho-[3',2',1':10,9,11]estr-4-en-3-one
(Z)-6'-(4-cyanophenyl)-9,11α-dihydro-17β-hydroxy-17α-[3-(1-oxo-3-methylbutoxy)-1-propenyl]-4'H-naphtho-[3',2',1': 10,9,11]estra-4,15-dien-3-one
(Z)-6'-(4-cyanophenyl)-9,11α-dihydro-17β-hydroxy-17α-[3-(1-oxo-2-methylpropoxy)-1-propenyl]-4'H-naphtho[3',2',1':10,9,11]estra-4,15-dien-3-one.

3. A compound according to claim 1, in which m has the value of 1.

4. A compound according to claim 1, in which m has the value of 2.

5. A compound according to claim 1, in which n has the value of 1.

6. A compound according to claim 1, in which n has the value of 0.

7. A compound according to claim 1, in which X is a nitrile group.

8. A compound according to claim 1, in which X is a fluorine atom.

9. A compound according to claim 1, having a single bond between carbon atoms 15 and 16.

10. A compound according to claim 1, having a double bond between carbon atoms 15 and 16.

11. A process for the preparation of steroid esters of general formula I according to claim 1, comprising esterifying a compound of formula II, which carries a free hydroxyl group and in which m, X and the dotted line between carbon atoms 15 and 16 have the meanings that are indicated in formula I, with an acid anhydride of formula III or an acid chloride of formula IV, in which n has the value of 0 or 1

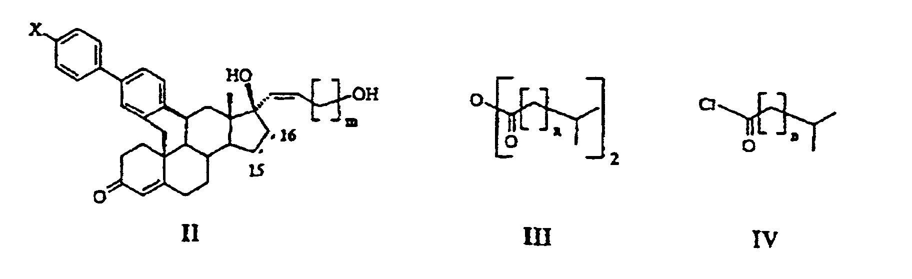

12. A pharmaceutical comprising at least one compound of general formula I according to claim 1 and a pharmaceutically acceptable vehicle.

13. The use of a compound of general formula I according to claim 1 for the production of a pharmaceutical.

## Revendications

1. Esters stéroïdiques de formule I 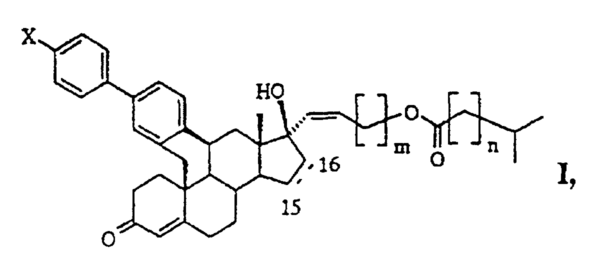 dans laquelle
m vaut 1 ou 2
n vaut 0 ou 1
X représente F ou CN et
la ligne en trait interrompu entre les atomes de carbone 15 et 16 symbolise la présence éventuelle d'une double liaison,
à l'exception du composé (Z)-6'-(4-cyanophényl)-9,11α-dihydro-17β-hydroxy-17α-[3-(1-oxo-2-méthylpropoxy)-1-propényl]-4'*H*-naphte[3',2',1':10,9,11]estr-4-én-3-one.

2. (Z)-6'-(4-cyanophényl)-9,11α-dihydro-17β-hydroxy-17α-[3-(1-oxo-3-méthylbutoxy)-1-propényl]-4'*H*-naphto[3',2',1':10,9,11]estr-4-én-3-one
(Z)-6'-(4-cyanophényl)-9,11α-dihydro-17β-hydroxy-17α[4-(1-oxo-2-méthylpropoxy)-1-butényl]-4'*H*-naphto[3',2',1':10,9,11]estr-4-én-3-one
(Z)-6'-(4-cyanophényl)-9,11α-dihydro-17β-hydroxy-17α[4-(1-oxo-3-méthylbutoxy)-1-butényl]-4'*H*-naphto[3',2',1':10,9,11]estr-4-én-3-one
(Z)-9,11α-dihydro-6'-(4-fluorophényl)-17β-hydroxy-17α[4*-*(1-oxo-3-méthylbutoxy)-1-butényl]-4'*H*-naphto[3',2',1':10,9,11]estr-4-én-3-one
(Z)-6'-(4-cyanophényl)-9,11α-dihydro-17β-hydroxy-17α[3-(1-oxo-3-méthylbutoxy)-1-propényl]-4'*H*-naphto[3',2',1':10,9,11]estra-4,15-dién-3-one
(Z)-6'-(4-cyanophényl)-9,11α-dihydro-17β-hydroxy-17α[3-(1-oxo-2-méthylpropoxy)-1-propényl]-4'*H*-naphto[3',2',1':10,9,11]estra-4,15-dién-3-one

3. Composés selon la revendication 1, dans lesquels m vaut 1.

4. Composés selon la revendication 1, dans lesquels m vaut 2.

5. Composés selon la revendication 1, dans lesquels n vaut 1.

6. Composés selon la revendication 1, dans lesquels n vaut 0.

7. Composés selon la revendication 1, dans lesquels X désigne un groupe nitrile.

8. Composés selon la revendication 1, dans lesquels X désigne un atome de fluor.

9. Composés selon la revendication 1, dans lesquels une liaison simple se trouve entre les atomes de carbone 15 et 16.

10. Composés selon la revendication 1, dans lesquels une double liaison se trouve entre les atomes de carbone 15 et 16.

11. Procédé de préparation d'esters stéroïdiques de formule générale I selon la revendication 1, **caractérisé en ce que** des composés de formule II portant un groupe hydroxy libre et dans laquelle m, X et la ligne en trait interrompu entre les atomes de carbone 15 et 16 ont les significations indiquées à la formule I, sont estérifiés avec un anhydride d'acide de formule III ou un chlorure d'acide de formule IV, dans lesquelles n vaut 0 ou 1.

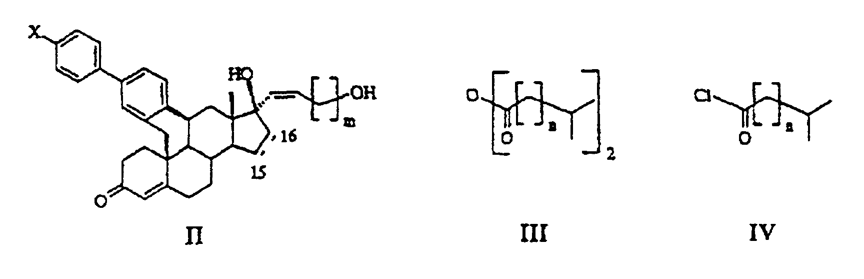

12. Médicament comprenant au moins un composé de formule générale I selon la revendication 1 ainsi qu'un support pharmaceutiquement acceptable.

13. Utilisation des composés de formule générale I selon la revendication 1 pour la préparation de médicaments.
